(19) Europäisches Patentamt
European Patent Office
Office européen des brevets

(11) Publication number: 0 161 073
A2

# (12) EUROPEAN PATENT APPLICATION

(21) Application number: 85302457.8

(22) Date of filing: 04.04.85

(51) Int. Cl.[4]: A 61 K 7/13
D 06 P 1/32

(30) Priority: 09.04.84 US 598466
22.10.84 US 664113

(43) Date of publication of application: 13.11.85 Bulletin 85/46

(84) Designated Contracting States: BE CH DE FR GB IT LI NL SE

(71) Applicant: REPLIGEN CORPORATION
101 Binney Street
Cambridge Massachusetts 02142(US)

(72) Inventor: Herlihy, Walter C.
648 Huron Avenue
Cambrige Massachusetts 02138(US)

(74) Representative: Perry, Robert Edward et al,
GILL JENNINGS & EVERY 53-64 Chancery Lane
London WC2A 1HN(GB)

## (54) Dyeing composition and its use in dyeing hair.

(57) A dye composition comprises (1) a suitable organic compound to assist dye penetration, (2) a dye precursor which is dopamine, D-dopa, L-dopa, D,L-dopa or an analogue thereof, and (3) iodate or periodate. The composition can be used to colour hair or other keratinous fibres, e.g. from gray to black.

EP 0 161 073 A2

This invention relates to a dye composition and its use in dyeing hair.

In the past thirty years, a variety of chemical systems for dyeing hair has been developed. The most commercially successful system uses phenylenediamine combined with various couplers and modifiers, and hydrogen peroxide as an oxidant. Although this system covers gray hair well, it suffers from the problems that phenylenediamine is a known sensitiser, that the hair is damaged by repeated exposure to alkaline peroxide, and that the colour produced may fade with time, to an off-shade. Further, Crevelli et al, Fd. Cosmet. Toxicol. 19 (1981) 79-84, disclose that p-phenylenediamine is oxidised in the presence of resorcinol by hydrogen peroxide to a mutagen that is percutaneously absorbed.

US-A-4021538 discloses a method for producing pigmentation in hair and skin; the method uses esters of dopa, because of their solubility in both water and lipid solvents. Dopa itself is practically insoluble in water or lipid solvents. Thus, when topically applied, it will penetrate the hair only slightly. US-A-4390341 discloses a composition for colouring hair or skin which employs, inter alia, acylated dopamine or acylated tyrosine derivatives and omega-amino-acids.

The need exists for an effective dye composition to change grey hair to a pleasing black, brown or other colour. Such a dye composition desirably should be non-nutagenic, non-sensitising, do little or no damage to the hair and skin, and impart a desired colour which is stable to repeated washings and weathering.

A dye composition according to the present invention comprises (1) a suitable organic compound to assist dye penetration; (2) a dye precursor such as dopamine, D-dopa, L-dopa or D,L-dopa; and (3) iodate or periodate. The result of this combination of dyeing elements is manifested by pleasing and stable colours imparted to hair and other keratinous fibres. For example, gray hair is dyed to a pleasing and stable red, brown or black colour.

The dyeing agent is non-sensitising and non-mutagenic. In addition, a melanin-like dye confers several surprising advantageous

properties to the hair. There is minimal or no damage to the hair and no dyeing of the skin. The colour is stable to washing but, after a large number (e.g. 20) of washings or after permanent waving, the colour may fade "on shade", e.g. to a lighter tone of the same colour, thus avoiding the red or green overtones which are often observed after repeated washing or perming of hair coloured with commercially-available phenylenediamine-based dyes.

Another unforeseen advantageous property of the novel composition is protection against damage by ultraviolet light; by comparison, irradiation of gray hair, natural or dyed with a commercial dye, causes damage.

Many compounds are known in the hair dyeing art, which can be used to assist dye penetration. A suitable organic compound for use in the invention is compatible with the dye precursor and does not react with the oxidant. Examples of suitable organic compounds are phenols, including thymol (2-isopropyl-5-methylphenol); $C_{5-10}$ ketones, including acetophenone, 4-ethylacetophenone, cyclohexanone, 4,5-dimethylacetophenone, 3,5-dimethylcyclohexanone and 4-methylcyclohexanone; $C_{5-10}$ esters, including ethyl benzoate, benzyl acetate, benzyl propionate and benzyl butyrate; alcohols, including (a) $C_{5-10}$ carbocyclic alcohols such as cyclohexanol and 2-methylcyclohexanol, (b) heterocyclic alcohols such as furfuryl alcohol, (c) $C_{5-8}$ aliphatic alcohols such as hexanol and 2-methyl-1-pentanol, and (d) aryl alkanols such as benzyl alcohol, α,α-dimethylbenzyl alcohol, α-propylbenzyl alcohol, DL-α-methylbenzyl alcohol, 2-benzyloxyethanol, 2-benzyloxypropanol, 2-benzyloxybutanol, and ethylene glycol phenyl ether; lactones, comprising butyrolactone; 1,2-propylene glycol carbonate; ethylene carbonate; tetramethylene sulfone; butadiene sulfone; tetrahydro thiophene dioxide; 1-substituted azacycloalkane-2-ones, comprising 1-n-dodecylazacycloheptan-2-one; and ethylene glycol sulfite. The suitable organic compound may be present at a concentration of 0.1-30%, and be a mixture of two or more compatible compounds.

The second key element in the dyeing combination of the subject invention is the use of an acceptable dye precursor, for example, dopamine, D-dopa, L-dopa, D,L-dopa, or suitable analogs thereof, at a concentration of about 1 to about 100 mg/ml, preferably about 5 to about 25 mg/ml, to the dye composition. The dye precursor, which can be a mixture of two or more compatible compounds, can be shown by the formula:

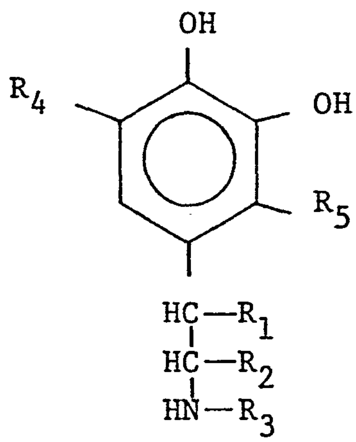

wherein

$R_1$, $R_2$ can be the same or different and are: H, alkyl (1-4C), $NH_2$, OH, COOR' (R' is alkyl 1-4C or H), $CONH_2$, halogen (Cl, Br, I, F), OR" (R" is alkyl 1-4C); $CH_2OH$, $CH_2NH_2$, or CONR'R" wherein R' and R" can be the same or different;

$R_3$ is H or alkyl (1-4C);

$R_4$, $R_5$ can be the same or different and are: H, alkyl (1-4C), $NH_2$, OH, COOR', $CONH_2$, halogen, OR", $NO_2$, $SO_3$, NHR", NR"R", $CH_2OH$, or $CH_2NH_2$.

Preferred dye precursors can be shown by the formula

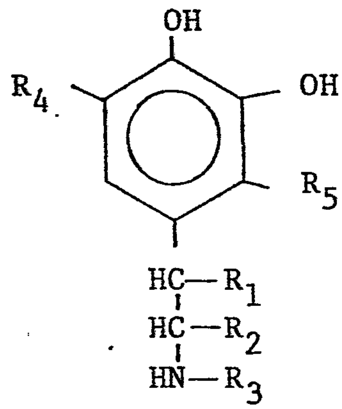

wherein

$R_1$, $R_2$ can be the same or different and are: H, $CH_3$, $C_2H_5$, OH, or halogen;

$R_3$ is H, $CH_3$, or $C_2H_5$;

$R_4$, $R_5$ can be the same or different and are as defined previously.

The most preferred dye precursor is dopamine, where in the above formula $R_1=R_2=R_3=R_4=R_5=H$.

Examples of suitable analogs of dopamine, D-dopa, L-dopa, or D,L-dopa are 2-methyldopamine, 5-methyldopamine, or α-(aminomethyl)-3,4-dihydroxybenzyl alcohol. A wider variety of hair colors can be obtained, if desired, by use of dopamine, D-dopa, L-dopa, D,L-dopa, or a suitable analog as defined herein, in conjunction with a color modifier. Such a color modifier, at a concentration of about 0.1 to about 10 mg/ml of the dye composition, can be, for example, 4-methylcatechol, 3,4-dihydroxybenzoic acid, 3,4-dihydroxybenzaldehyde, 4-bromocatechol, catechol, 4-methoxycatechol, and the

like; or substituted phenols or anilines, for example m-aminophenol, resorcinol, and the like; a thiol such as a cysteine, glutathione or mercaptoacetic acid; or an amino acid such as lysine. The color modifier can be a mixture of two or more compatible compounds.

The third key element is an iodate or periodate oxidizer at a concentration of about 1 to about 50 mg/ml. Though the sodium salt form is preferred, any alkali metal or alkaline earth metal salt of iodate or periodate can be used.

If desired, a thickening agent can be incorporated into the dyeing combination to minimize dripping from the hair. Suitable thickeners include water-soluble resins and gums, for example, carrageenan, guar gum, locust bean gum, and the like; also polymers, such as Carbopol 934 (BF Goodrich Chemical Group, Cleveland, OH), polyvinyl alcohol, and the like; or inorganic materials, for example, Veegum (Vanderbilt Company, Norwalk, CN), nonoxynol-4, nonoxynol-9 (Heterene Chemical Co., Paterson, NJ) or combinations of nonoxynols, and the like. A particularly useful thickening agent is polyethylene glycol-150 distearate (PEG-150 distearate) (Heterene). For example, a 10% solution of PEG-150 distearate is compatible with the dyeing reaction and gives a commercially useful thickened product. Thickening agents can be used in a variety of combinations. The thickener can be present at about 0.1 to about 25% by weight, relative to the total weight of the dye composition.

Upon contacting gray hair with a dye composition comprising an organic compound to assist dye penetration, dopamine, D-dopa, L-dopa, D,L-dopa, or a suitable analog, and an oxidizing agent, at a pH of 3 to 7 there is obtained hair having a pleasing stable black color. Other colors can be obtained by use of color modifiers and persulfate, as disclosed herein. When

a brownish color is desired for the gray hair, then the addition of a persulfate, for example, potassium persulfate at a concentration of about 0.1 to about 20 mg/ml, preferably about 1 to about 5 mg/ml, to the dye composition, can be added.

When an organic compound or color modifier, defined above, is not readily soluble in aqueous solution, it can be solubilized with up to about 50% of an organic solvent, for example, propanol, ethanol, isopropyl alcohol, and the like. These solubilizing solvents are not suitable for use to assist dye penetration.

The pH of the dye composition should be from about 3 to about 7.0. Standard buffers and acidifying agents can be used to maintain the pH within this range by procedures well known in the hair dye art.

The dyeing process is enhanced at temperatures above room temperature. For example, better dye penetration is realized at 35°C than at 20°C. Higher temperatures can be achieved by a variety of methods, including covering the head with a plastic cap during the dyeing reaction; warming of the dye precursor and oxidant solutions before application, either with an external device or with an exothermic reaction initiated by mixing of, for example, the precursor and oxidant solutions; with an infrared lamp; or by other means well known in the hair dyeing art.

Anionic, cationic, non-ionic or amphoteric water-soluble surface-active agents also can be included in the dye composition. Surface-active agents that can be used are alkylbenzenesulfonates, alkylnaphthalenesulfonates, sulfates, ether-sulfates, and sulfonates of fatty alcohols, quaternary ammonium salts, such as trimethylacetylammonium bromide and cetylpyridinium bromide, diethanolamides of fatty acids, and polyoxyethyleneated or polyglycerolated acids, alcohols or alkylphenols. The surface-active agents are preferably

present in the dye composition in a proportion of about 0.1 to about 15% by weight.

Advantageously, a stabilizer can be added to the dye composition if the dopamine dye precursor is in the dissolved state. A suitable stabilizer is the reducing agent sodium metabisulfite at a concentration of 1 to 5 mM.

The subject dye composition can be used in various forms, for example, liquid, cream, gel or an aerosol, or in any other form that is suitable for dyeing hair and other keratinous fibers.

Following are examples that illustrate products of the invention and procedures for practising the invention. These examples should not be construed as limiting. All percentages are by weight and all solvent mixture proportions are by volume unless otherwise noted.

Example 1

Gray hair is contacted with a 12% solution of isopropyl alcohol comprised of dopamine at a concentration of about 5 to about 25 mg/ml, about 10% of ethylene glycol phenyl ether, and sodium periodate at a concentration of about 2 to about 10 mg/ml. The dyeing process proceeds for about 20 to about 60 min, after which the dyed hair is rinsed. The hair now has a pleasing black color.

Example 2

Gray hair is contacted with an aqueous solution comprised of dopamine at a concentration of about 2 to about 10 mg/ml, catechol at a concentration of about 0.4 to about 2 mg/ml, about 2 to about 4% of benzyl alcohol, the known thickener carrageenan at about 0.5 to about 5% by weight, sodium iodate at a concentration of about 1 to about 5 mg/ml and ammonium persulfate at a concentration of about 1 to about 5 mg/ml. The process

proceeds for about 20 to about 60 min, after which the dyed hair is rinsed. The hair now has a pleasing auburn color.

Example 3

Gray hair is contacted with a 10% solution of isopropyl alcohol comprised of dopamine at a concentration of about 5 to about 25 mg/ml, about 1 to about 2% of thymol, sodium iodate at a concentration of about 2 to about 10 mg/ml, and ammonium persulfate at a concentration of about 0.5 mg/ml. The process proceeds for about 20 to about 60 min, after which the dyed hair is rinsed. The hair now has a pleasing black color.

Example 4

Gray hair is contacted with an aqueous solution comprised of D,L-dopa or dopamine, at a concentration of about 5 to about 25 mg/ml, 4-methyl catechol in a concentration of about 2 to about 10 mg/ml, about 2 to about 4% of benzyl alcohol, the known thickener carrageenan at about 0.5 to about 5% by weight and sodium iodate at a concentration of about 2 to about 10 mg/ml. The process proceeds for about 20 to about 60 min, after which the dyed hair is rinsed. The hair now has a pleasing brown color. D-dopa or L-dopa may be used instead.

Example 5

Upon adding potassium persulfate to the dye composition of Example 1, at a concentration of about 1 to about 5 mg/ml of potassium persulfate to the dye composition, there is obtained hair with a brownish color.

Example 6

Gray hair is contacted with an aqueous solution comprised of dopamine at a concentration of about 5 to about 10 mg/ml, cysteine at a concentration of about 5 to about 10 mg/ml, about 4% benzyl alcohol, about 1 mg/ml ammonium persulfate and about 2 to about 5 mg/ml sodium iodate. The dyeing reaction proceeds for about 20 to about 60 min, after which the hair is rinsed.

The hair has a red-orange colour.

Example 7

Gray hair is contacted with an aqueous solution of 10% polyethylene glycol-150 distearate, 5 mg/ml dopamine hydrochloride, 22 mg/ml sodium iodate and 20% ethylene carbonate. The scalp is covered with a plastic cap and the reaction is allowed to proceed for 20 to 60 min, after which the hair is rinsed. The hair now has a pleasing black colour.

Example 8

Gray hair is contacted with an aqueous solution of 10% polyethylene glycol-150 distearate, 4 mg/ml dopamine hydrochloride, 0.4 mg/ml lysine hydrochloride, 20 mg/ml sodium iodate and 20% propylene carbonate. The scalp is covered with a plastic cap and the reaction is allowed to proceed for 20 to 60 min, after which the hair is rinsed. The hair now has a pleasing brown colour.

Example 9

Oriental gray hair is contacted with an aqueous solution containing 5 mg/ml dopamine hydrochloride, 11 mg/ml sodium iodate and 30% butadiene sulfone. The scalp is covered with a plastic cap and the reaction is allowed to proceed for 20 to 60 min, after which the hair is rinsed. The hair now has a pleasing black colour.

White (rather than gray) hair, and other keratinous fibres, may be coloured by the procedure of, say, Examples 1 to 5, to give the same final colour.

CLAIMS

1. A dye composition which comprises

(1) an organic compound which assists dye penetration;

(2) a dye precursor of the formula

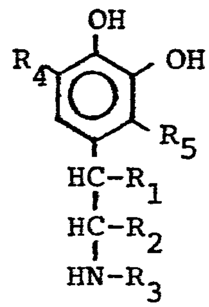

wherein $R_1$ and $R_2$ are independently selected from H, $C_{1-4}$ alkyl, $NH_2$, OH, COOH, ($C_{1-4}$ alkoxy)carbonyl, $CONH_2$, Cl, Br, I, F, $C_{1-4}$ alkoxy, $CH_2OH$, $CH_2NH_2$, ($C_{1-4}$ alkyl)aminocarbonyl and di($C_{1-4}$ alkyl)aminocarbonyl; $R_3$ is H or $C_{1-4}$ alkyl; and $R_4$ and $R_5$ are independently selected from H, $C_{1-4}$ alkyl, $NH_2$, OH, COOH, ($C_{1-4}$ alkoxy)carbonyl, $CONH_2$, Cl, Br, I, F, $C_{1-4}$ alkoxy, $NO_2$, $SO_3$, ($C_{1-4}$ alkyl)amino and di($C_{1-4}$ alkyl)amino; and

(3) an iodate or periodate oxidiser.

2. A dye composition according to claim 1, wherein $R_1$ and $R_2$ are independently selected from H, $CH_3$, $C_2H_5$, OH, Cl, Br, I and F; and $R_3$ is H, $CH_3$ or $C_2H_5$.

3. A dye composition according to claim 1, wherein the dye precursor is dopamine, D-dopa, L-dopa or D,L-dopa.

4. A dye composition according to any preceding claim, wherein the organic penetrant is selected from phenols, including thymol; $C_{5-10}$ ketones, including acetophenone, 4-ethylacetophenone, cyclohexanone, 2,4-dimethylacetophenone, 3,5-dimethylcyclohexanone and 4-methylcyclohexanone; $C_{5-12}$ esters, including ethyl benzoate, benzyl acetate, benzyl propionate and benzyl butyrate; alcohols such as (a) $C_{5-10}$ carbocyclic alcohols, including cyclohexanol and 2-methylcyclohexanol, (b) heterocyclic alcohols, including furfuryl alcohol, (c) $C_{5-8}$ aliphatic

alcohols, including hexanol and 2-methyl-1-pentanol, and (d) aryl alkanols, including benzyl alcohol, α,α-dimethylbenzyl alcohol, α-propylbenzyl alcohol, DL-α-methylbenzyl alcohol, 2-benzyloxyethanol, 2-benzyloxypropanol, 2-benzyloxybutanol and ethylene glycol ether; lactones, including butyrolactone; 1,2-propylene glycol carbonate; ethylene carbonate; tetramethylene sulfone; butadiene sulfone; tetrahydrothiophene dioxide; 1-substituted azacycloalkane-2-ones, including 1-n-dodecylazacycloheptan-2-one; and ethylene glycol sulfite.

5. A dye composition according to claim 4, wherein the organic penetrant is benzyl alcohol or ethylene carbonate.

6. A dye composition according to any preceding claim, which comprises from 0.1 to 30% of the organic penetrant, from 1 to 100 mg/ml of the dye precursor, and from 2 to 10 mg/ml of the oxidiser.

7. A dye composition according to any preceding claim, which additionally comprises a thickener, preferably polyethylene glycol-150 distearate.

8. A dye composition according to any preceding claim, which additionally comprises a colour modifier, preferably catechol or cysteine.

9. A dye composition according to any preceding claim, which additionally comprises a persulfate and/or a stabiliser.

10. A process for dyeing hair and other keratinous fibres, which comprises applying thereto a dye composition according to any preceding claim, and rinsing the dyed hair or fibres.